# EUROPEAN PATENT APPLICATION

(11) **EP 0 643 038 A2**
(43) Date of publication of application: **15.03.1995**
(21) Application number: 94201943.1
(22) Date of filing: 06.07.1994
(51) Int. Cl.: C07C 219/06, C07C 229/16, C07C 233/36, C07C 233/38, B01J 2/30, C11D 1/62, C23F 11/14, C08L 95/00, B01F 17/00, C11D 1/46, D06M 13/342, D06M 13/224, D06M 13/325, D06M 13/46, A01N 37/12, A01N 37/20, A01N 37/44, A61K 7/08

(54) **Novel polyfunctional cationic surface active agents, compositions comprised thereof, process for the preparation thereof and uses**

(30) Priority: 15.07.1993 ES 9301594
(71) Applicant: ARANOR, S.A., E-50687 Isuerre (Zaragoza) (ES)
(72) Inventor: The designation of the inventor has not yet been filed
(74) Representative: Ungria Lopez, Javier

(57) **Abstract**

Surface active agents: (I) to (X) wherein R and R' = alkyl, akylaryl or alkoxyalkyl; R₁ = H, OH; R₂ and R₃ = benzyl, alkyl or oxyalkyl; X = Cl⁻, SO₄CH₃⁻ and SO₄C₂H₅⁻; s = 2 and 3; and n and m = 1 to 5.

Processes: (a) reacting a N,N-dialkyl-N',N'-dipolyalkoxy-1, s-alkanodiamine with monocarboxylic acid to give (II) or (IV), which by alkylation provide (I) and (III), (b) reacting a N,N-dialkyl-N',N'-dipolyalkoxy-1, 2-alkanodiamine with methyl acrylate and a fatty acid to give (VI) or (VII), which by alkylation provide (V) and (VII); (c) reacting a N,N-dialkyl-1,s-alkanodiamine with a carboxylic acid and reacting the resulting amide with glyceric chloroester to give (IX); (d) reacting a N,N-dialkyl-(2-acryloxyethyl)amine with a fatty acid to give an intermediate which by reaction with glyceric chloroester gives (X).

Compositions: variable proportions of (I) to (X) together with other components.

Use: Fabric softeners and hair conditioners, disinfectants, anticaking agents, asphaltic emulsifiers, etc.

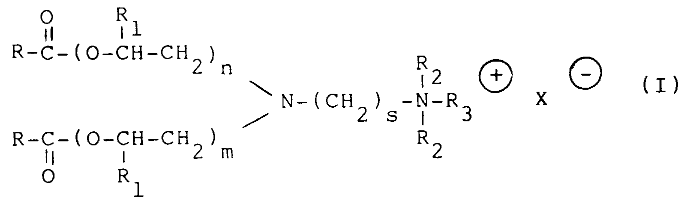

## Description

### TECHNICAL FIELD OF THE INVENTION

The present invention fits within the technical field of surface active agents and specifically refers to novel polyfunctional cationic surface active agents that combine the softening and wetting effects along with the capacity of biodegradation which makes them extremely attractive from the point of view of consumption and marketing.

### PRIOR ART OF THE INVENTION

Over the last few years important advances have been made in the formulation of household softeners, up to the point that before 1990 the market that could be considered static suddenly began to move with very important dynamics.

On the other hand, the interest of offering concentrated products, mainly ones that can be diluted with three times their volume in water, that do not gellify during storage, which dilute instantly without forming lumps and also with a stable viscosity until they are all used up is obvious. Simultaneously, the concentrated product can also be rapidly and easily added to the washing machine at the end of the washing cycle.

The greater and greater acceptance of concentrates has commercial and ecological advantages. Hence, the reduction of the cost of the container and reduction of pollution caused by the container above all in the case of brick-shaped cardboard containers are worth pointing out. Besides, the reduction of space on store shelves and the smaller transporting of water and weight by housewives is also worth pointing out.

On the other hand, a very important factor that has influenced research on new chemical structures, recently introduced in the corresponding formulations, is the need to offer molecules with a greater biodegradability.

As a general rule, it is accepted that a cationic structure, in order to be used as a softener base, must proferably be comprised of two fatty chains of C₁₀-C₂₂ carbon atoms and two short chains of the methyl, hydroxyethyl type, etc., also establishing a ratio between the insaturation of the chain and the balance between the softening power of the wetting power of the product. A complement of this general rule is the fact that upon introducing labile functional groups, especially of the ester group, the biodegradability of the molecule increases very remarkably.

In favor of this latest tendency is also the fact of the stability of these molecules against hydrolysis in normal working conditions.

The number of publications that cited molecules oriented in the above cited line already begins to be considerable and among said works we can cite:

U.S. patent no. 3,915,867 of Kang et al., of October 1975, wherein the obtainment of cationic derivatives of triethanolamine esters is described.

U.S. patent no. 4.039.565, of Throckmorton et al., of August 1977, wherein the obtainment of cationic products of functional etheramide groups is set forth.

U.S. patent no. 4.038.294 of Van Dyk et al., of 1977, wherein the use, in cosmetic preparations, of polyfunctional cationic derivatives, containing esteramide groups is described.

U.S. patent no. 4.370.272 of Wechester et al. of 1983, wherein the use of the above compounds in the field of household softeners is described.

U.S. patent no. 4.439.331 of Billenstein et al. of March 1984 wherein the use of cationic derivatives containing diamine esters is described.

U.S. patent no. 4.540.521 of Garst et al., of September 1985, wherein the use of cationic derivatives obtained by quaternization of etheramines and triethanolamine as antistatic agents for fibers, is described.

U.S. patent no. 4.720.383 of Drach et al., of January 1988, wherein the use of imidazolinic cationic derivatives containing functional amidoester groups is described.

European patent no. 0.293.955 of Walley et al., of December 1988, wherein the use of cationic derivatives obtained by esterification of methylisopropanolamines and acid chlorides is described.

European patent no. 0.295.739 of Change et al., of December 1988, which follows the line of the previous patent starting with methyl, alkylethanolamines.

Within this general context, the application has focused his research and has discovered novel molecules that combine the softening effects and wetting effects together with a biodegradation capacity that makes them prefectly attractive from a point of view of consumption and marketing.

With all this, the applicant considers that the molecules described in the present specification are an important contribution to the group of products that, having a good performance of use as softeners, they can be included in the field of products highly respectful of the environment, especially in the case of molecules that are presented in the form of amine salts.

The advantages of the products of the present invention are applicable, not only to the field of household softeners (perhaps the most attractive one due to their marketing volume), but also to other markets in which the applicant considers it convenient to divulge the use thereof.

### DETAILED DESCRIPTION OF THE INVENTION

Just as is indicated in the title, the present invention refers to novel polyfunctional cationic surface active agents, as well as to compositions that contain them, to the processes for the preparation thereof and to the uses thereof.

In the present specification, a series of novel compounds with one or two aliphatic C₆-C₂₄ long chains containing one ester or amide group and that have one or two nitrogen atoms in the form of a secondary or tertiary amine are made known. These aminic functions can be in a free form or in the form of salts or acids such as hydrochloric, acetic, formic acid, etc., or in the form of quaternary ammoniums obtained by the normal quaternization methods with agents such as methyl chloride, methyl sulfate, diethyl sulfate, etc., or else in the form of amphoteric surface active agents after reacting with sodium monochloroacetate, acrylates, etc.

Specifically, the novel surface active agents of the present invention have the following structural formulae: 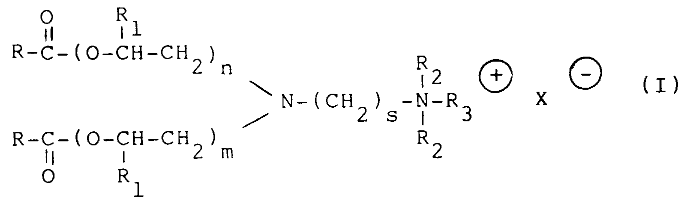 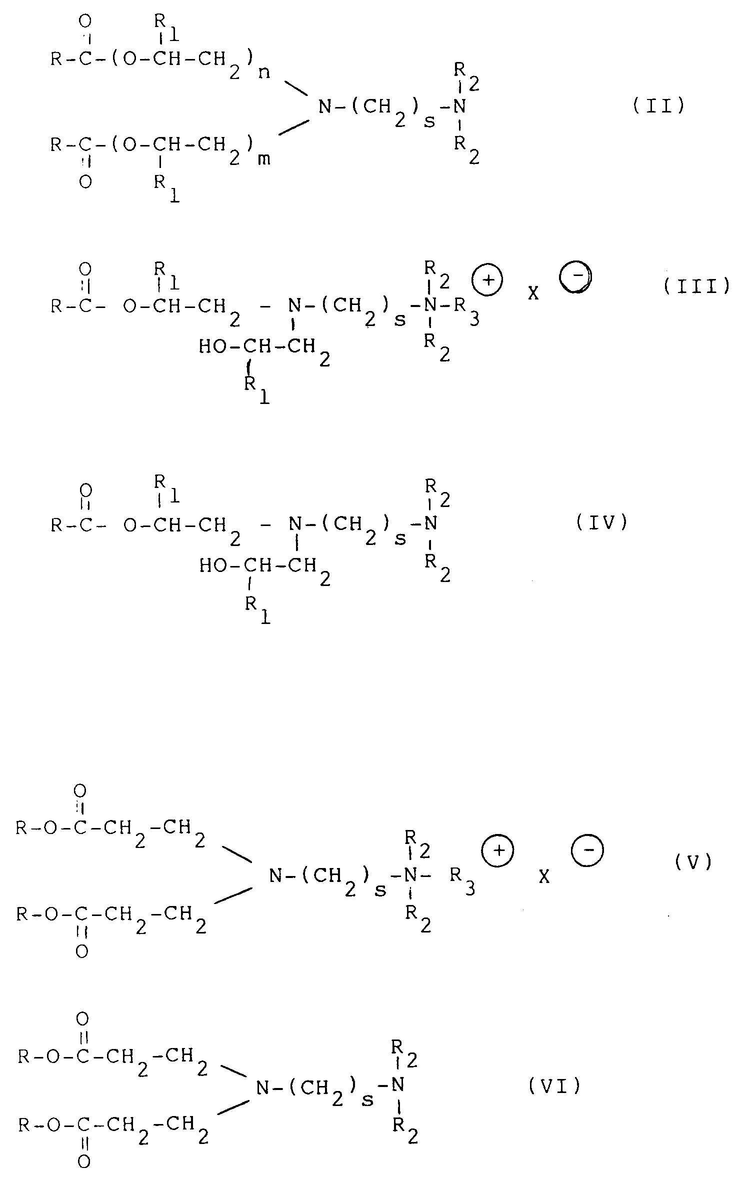 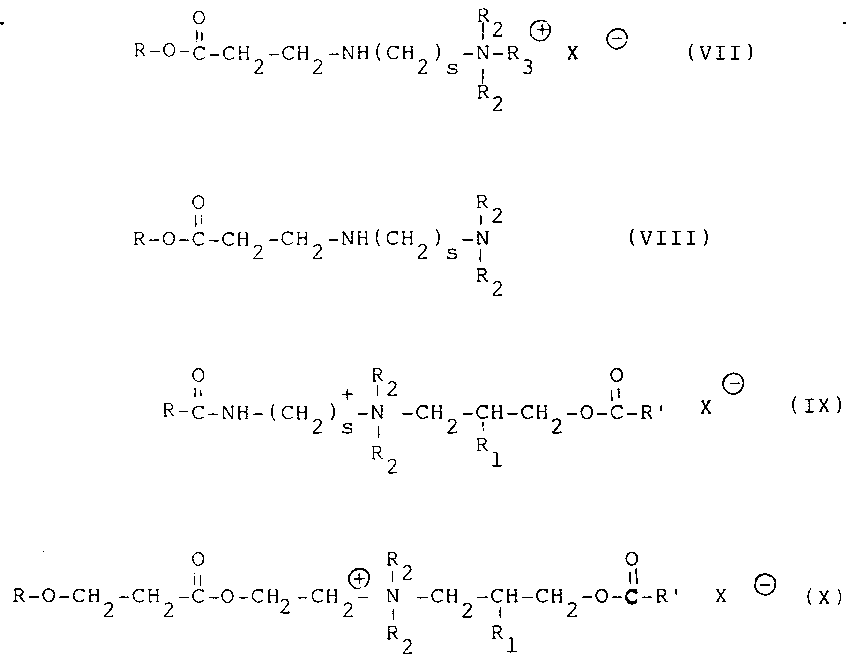
wherein:
R and R' represent alkyl, alkylaryl or alkoxyalkyl radicals, wherein the alkyl residue can be linear or branched and contains from 6 to 24 carbon atoms;
R₁ represents hydrogen or hydroxyl;
R₂ and R₃ represent a benzyl, alkyl or oxyalkyl group, wherein the alkyl residue contains from 1 to 6 carbon atoms;
X^{⊖} represents a Cl^{⊖} SO₄CH₃^{⊖} or SO₄C₂H₅^{⊖} anion
n and m represent an integer between 1 and 5, both inclusive; and
s represents an integer selected between 2 and 3
The compounds of formulae (I) and (X) mentioned above can be obtained in accordance with the following

### REACTION SCHEMES

### Type 1 Reaction

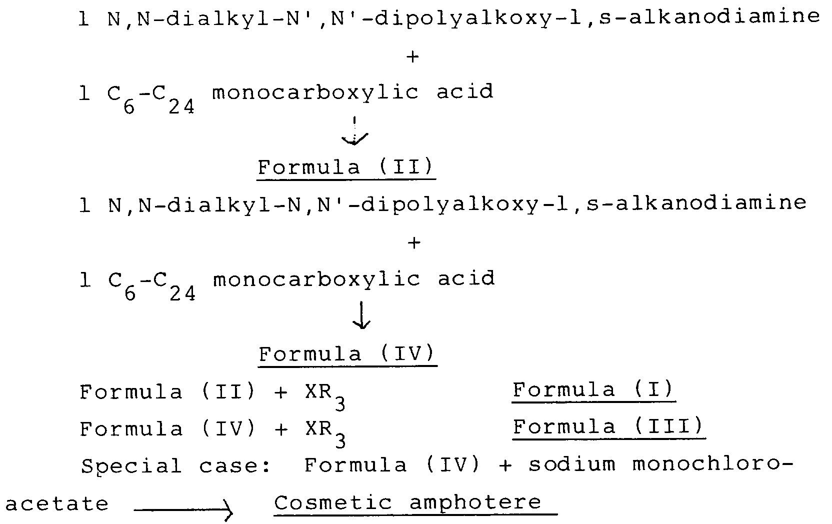

### Type 2 Reaction

1 N,N-dialkyl-1,s-alkanodiamine + 2 methyl acry late + 2 fatty alcohol → Formula (VI)

1 N,N-dialkyl-1,s-alkanodiamine + 1 methyl acry late + 1 fatty alcohol → Formula (VIII)

Formula (VI) + XR₃ → Formula (V)

Formula (VIII) + XR₃ → Formula (VII)

Special case: Formula (VIII) + sodium monochloro acetate → Cosmetic amphotere

### Type 3 Reaction

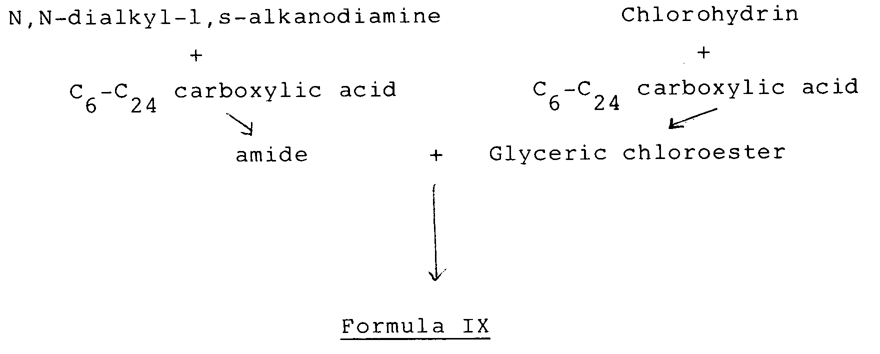

### Type 4 Reaction

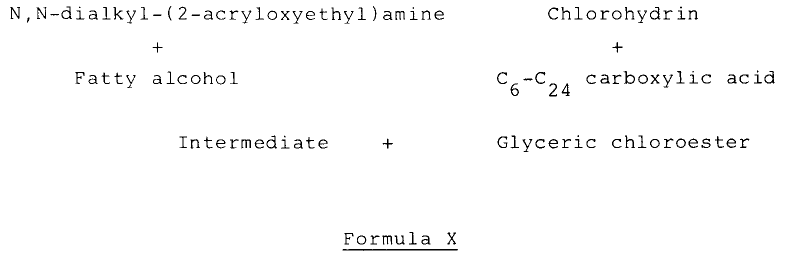

The different reactions implied in the above scheme will be explained hereinafter.

### Type 1 Reaction

### Step A)

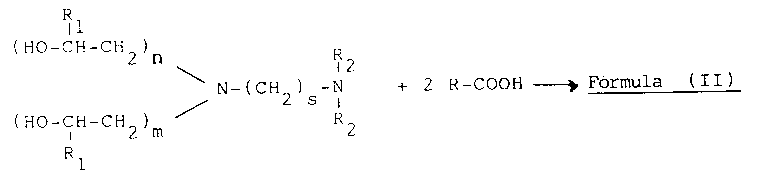

This step consists of the esterification of a N,N-dialkyl-N'N'-dipolyalkoxy-1,s-alkanodiamine with two moles of monocarboxylic acid containing from C₆ to C₂₄ carbon atoms, resulting a tertiary diamine diester. The reaction can likewise be carried out with the corresponding metyl esters. The reaction in the case of the acid takes place at temperatures of 120-200^{º} C, preferably at 140-180^{º} C, using classic acid catalysts such as, for example, p-toluenesulfonic acid. The acid/amine molar ratio is normally 2:1 whereby a well defined structure is achieved, with the absence of practically all derivatives with a fatty chain or with three, so detrimental to the effects of use in the field of household softeners.

### Step B)

The compound obtained in previous step A is reacted with the stoichiometric amount of an alkylating agent such as methyl chloride or dimethyl sulfate or diethyl sulfate at temperatures between 40-90º C and in the presence of small amounts (from 5 to 25%) of a solvent of the type of methanol, ethanol, isopropanol, propyleneglycol, etc. In this way the compound of formula (I) is obtained from the compound of formula (II.)

The compound of formula (IV) is likewise obtained by means of Type 1 reaction, Step A, but with an acid/amine molar ratio of 1:1, whereby the monoester, in other words, the corresponding compound with a single fatty chain instead of two is preferably obtained.

The compound of formula (III) is obtained from the compound of formula (IV) by quaternization of the same with methyl chloride or benzyl chloride depending on the field of use that is sought. The reaction conditions are likewise those described above.

A special case of the compound (III) is comprised by the derivative obtained by reaction of the compound of formula IV with sodium monochloroacetate in an aqueous medium heating for 3 to 8 hours at a temperature of 60-90º C and at a pH alkaline enough during the entire addition of the reagent for the purpose of achieving the carboxymethylation, obtaining an amphoteric molecule corresponding to formula (I) wherein R₃ = carboxymethyl very useful in cosmetic formulations such as shampoos and bath gels. The product obtained has a content of active product of 25-40%.

### Type 2 Reaction

### Step A)

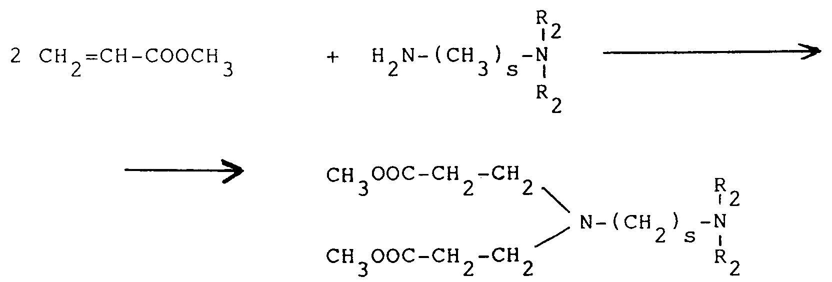

Two mols of methyl acrylate are reacted in this step, at a temperature between 25 and 60º C and for 3 to 12 hours with one mol of a N,N-dialkyl-1,s-alkanodiamine to give an intermediate product of formula:
(CH₃OOC-CH₂-CH₂)₂N-(CH₂)ₛ-N(R₂)₂ that is then subjected to the following step B.

### Step B)

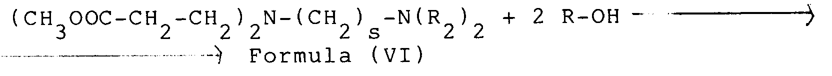

In this step, the intermediate product obtained in step A) is reacted, at a temperature of 100-120º C and a slight vacuum and in the presence of sodium methylate as catalyzed matter wit two mols of a fatty alcohol with elimination of two mols of methanol to produce the compound of formula (VI.)

### Step C)

The compound obtained in the above section, by means of a reaction identical to the one described in Step 1-B, can give rise to quaternary ammonium compounds of Formula V, in the same way as has been described above.

Similarly as that which has been described above, if the reaction 2-A is carried out with only one mol of methyl acrylate the monosubstituted derivative is preferably obtained than by subsequent reaction, according to Step 2-B, with one mol of fatty alcohol permitting the obtainment of the compound of Formula VIII, which, in turn, by subsequent reaction according to Step 2-C permitting the obtainment of the corresponding quaternary ammonium derivatives of Formula VII. The reaction can likewise be carried out with sodium monochloroacetate, as it has been described above, to obtain the molecules with amphoteric behaviour.

### Type 3 reaction

### Step A)

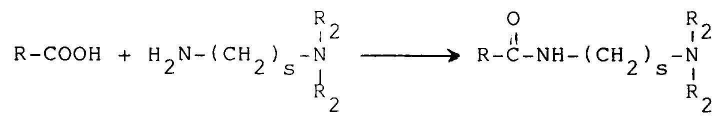

In this step, the reaction of amidation with a carboxylic acid with a chain of 6 to 24 carbon atoms with a N,N-dialkyl-1,s-alkanodiamine. The reaction takes place at a temperature of 130-200º C, preferably between 140 and 180º C with elimination of the water formed.

### Step B)

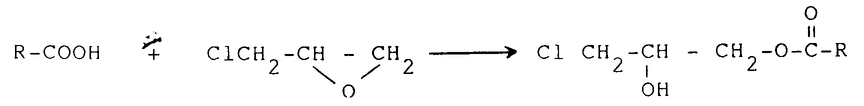

This reaction step is intermediate to obtain the glyceric chloroester which, in the following step, is reacted with the compound obtained in Step A. The reaction of a fatty acid with chlorohydrin takes place at a temperature between 80 and 120º C lasting 1 to 4 hours.

### Step C)

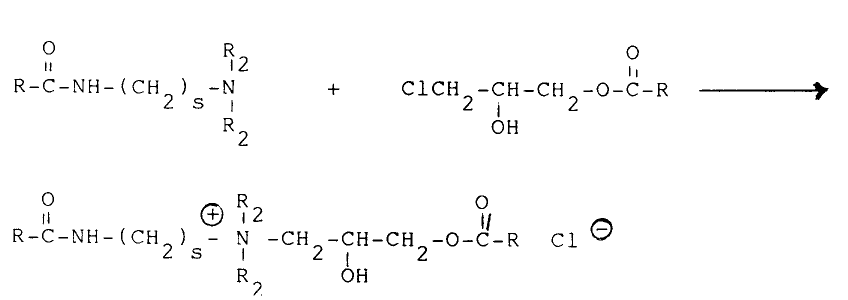

In this step of the reaction the condensation of the two intermediate molecules obtained in Steps B and C takes place, heating at a temperature of 60-90º C for 1 to 5 hours to produce the compound of Formula (IX) wherein R₁ is OH.

### Type 4 reaction

Step A) If for the use that is desired, it is convenient to eliminate the amide group of the molecule described in the above section a new synthesis reaction has been studied, just as is indicated below:

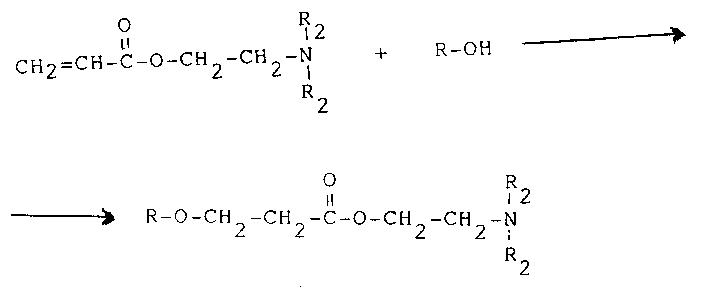

In this case a N,N-dialkyl-N-(2-acryloxyethyl)amine is reacted for 2 to 6 hours at a temperature of 50-80º C with one mol of a linear or branched fatty alcohol, whose chain cany vary between 8 and 20 carbon atoms, to give a compound with a formula similar to formulae IV to VIII, which must be also considered object of the present invention. Said compound is additionally subjected to the following transformation.

### Step B)

The compound resulting from above step A) is reacted with the intermediate compound described in reaction 3-B (in other words, the glyceric chloroester resulting from the reaction of 1 mol of fatty acid with 1 mol of epchlorohydrin), in accordance with the follwoing scheme: 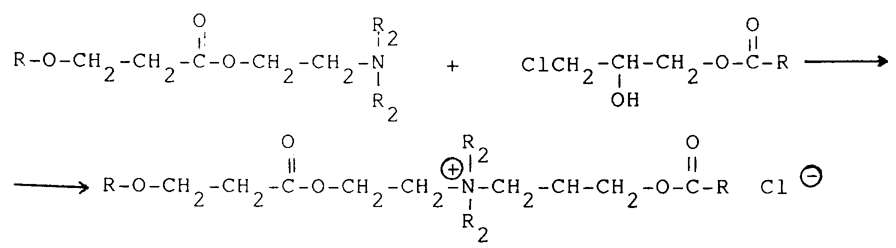
to produce the compound of formula (X).

The compounds of the above mentioned formulae (I) to (x) can be suitably formulated to obtain different types of compositions useable in multiple fields. These compositions also constitute one of the objects of the present invention. Hereinafter the most important ones are indicated:
1. The compounds of formulae I, II, V, VI, IX and X permit the formulation of good househould softeners used on cotton fabric and synthetic fibers, especially acrylic fibers. The formulations have a very good stability, a very good softening power, wetting power and biodegradability. Said formulations, in the form of an aqueous dispersion, can contain (the percentages are indicated in weight/weight), in general:
   1.1 Between 1% and 40% of product corresponding to the above cited formulae:
   1.2 Between 0% and 10% of a monohydric alcohol of C₁ to C₄ (ethane, isopropane, etc.) or a glycol of C₂ to C₆ (ethyleneglycol, propyleneglycol, etc.);
   1.3 Between 0% and 10%, preferably between 0% and 5% of a non-ionic surface active agent (ethoxylated alcohols, ethoxylated fatty amines, ethoxylated sorbitan esters, etc.) to facilitate dispersion and to improve stability;
   1.4 Between 90% and 60% of a solvent vehicle of the preparation, which is preferably water;
   1.5 Between 0 and 10% fatty acids, preferably hydrogenated tallow, and/or glyceryl monostearate;
   1.6 Other minor components such as:
      1.6.1 Generally known preservatives (formol, Kathon, Bronopol, etc.) in adequate amounts and with a maximum of 1 mg/kg, if necessary;
      1.6.2. Dyes as is usual;
      1.6.3 Compounds of a large variety of composition based on siloxanes, to provide better lubrication and wetting;
      1.6.4 Inorganic salts (NaCl, KCl, CaCl, MgCl, etc.) or cationic polymers with a high molecular weight, to adjust the final viscosity;
   1.7 Between 0% and 2% perfume, according to usual practice;
   1.8 The content of above point 1.1 of 1-40% of the compounds of the indicated formulae, can be shared with any other one of the quaternary ammonium derivatives known on the market, such as: dimethyldistearylammonium chloride, imidazolines, triethanolamine esterquate, etheramine cationic compound or the bases of all of them without quaternizing, etc.
      The Formulae I to X likewise permit the formulation of excellent hair conditioners that make hair silky, lubricous, easy to comb and with very good biodegradability. Said formulations (likewise in percentages weight/weight) generally in the form of aqueous dispersions can contain:
   1.9 Between 0.5% and 10% of the compound object of the invention;
   1.10 Between 0 % and 10% of a fatty alcohol generally of a saturated linear C₁₂-C₂₂ chain;
   1.11 Between 0% and 5% of cationic polymers described by the name CFTA, such as polyquaternium 7;
   1.12 Between 0% and 5% of non-ionic surface active agents of ethoxylated fatty alcohol, ethoxylated sorbitan ester types, etc.;
   1.13 Between 0% and 5% of other emollients such as paraffin oil, lanolin alcohol, etc.;
   1.14 Dyes, preservatives and perfumes, according to usual practice.

   The compounds of Formula III and VII, which regard to carboxymethylated derivatives, are compounds normally called "betaines" and which due to their amphoteric nmature are added to shampoos and bath gels for the purpose of reducing the irritant power of generally used anionic surface active agents and also to adjust the viscosity of said formulations.
2. The compounds of Formulae I, II, V, VI, IX and X formulated as such with a content of 0 to 20% of non-ionic surface active agent, generally of the family of ethoxylated fatty alcohols, generally with 15-25 mols of ethylene oxide, and in turn the mixture of the cited compounds with the described non-ionic compounds placed in an aqueous dispersion at a concentration of 5 to 50%, can be used to soften industrial fibers such as cotton, acrylic fibers, etc. The ideal dosage is that which permits a deposition of 0.05 to 0.2 % of the product on the fiber. Use thereof improves the touch and manageability in spinning, weaving, dyeing processes, etc.
   The compounds of Formulae III, IV, VII and VIII, especially in the case of containing C₁₂-C₂₀ chains provided with at least one double -CH=CH- bond, provide an excellent antistatic power to all natural and synthetic fibers improving the fiber/fiber and fiber/metal friction coefficients. In the case of short chains C₁-C₄ they are especially useful in open-end spinning processes.
   The Formulae III and VII, quaternized with benzyl chloride are useful as dyeing equalizers and retarders of acrylic fibers with cationic dyes. In the case of short chains, the products can be used as color strippers permitting the correction of unequal dyeing defects.
3. The compounds of Formulae III and VII, quaternized with benzyl chloride have excellent bactericide, fungicide and algicide power and they can be used for general disinfection processes and they can be effective at concentrations of 10 ppm.
   The compounds of Formulae I and V with short chains C₆-C₁₂ are useful in disinfectant formulations in animal farms, with the advantage over presently used quaternary ammonium derivatives (dimethyldodecylammonium chloride), due to their higher degree of biodegradability.
4. The compounds of Formulae IV and VIII without being neutralized or in the form of salts of formic, acetic acid, etc. behave as excellent anticaking agents for fertilizers (for example ammonium nitrate, potassium nitrate, etc.) , avoiding the absorption of moisture, the formation of dust and mainly caking of the granules during the storage time. The amount of amine salt used varies between 0.005 and 0.05% of the weight of the fertilizer. A mixture of between 10 and 50% of amine withoutr being neutralized and of 90-50% of a hydrocarbon instead of the above cited amine salt is also useful.
5. The compounds of Formulae IV and VIII have shown an excellent emulsifying power and offer a good potential in a abroad variety of emulsion systems, oil/water and water/oil. Hence they permit the preparation of asphaltic cationic emulsions that are usually formed (percentages in weight/weight) by 50-70% asphalt and 50-30% water containing the hydrochloride of the polyfuncitonal aminic compound in a proportion that can vary between 0.02 and 1%. The emulsion is obtained hot (asphalt at 125-130º C over water at 30-60º C) continuously and with the help of a colloidal mill.
   The emulsions obtained give good coating results over a large variety of aggregates and as to the results obtained they do not different from the classic ones as to adherence to wet surfaces, etc.
   In the case of working with more quaternized structures, shorter breakage times of the emulsions are achieved.
   The acetates of C₁₂-C₁₈ chain polyfunctional aminic structures are excellent corrosion inhibitors for iron and steel materials.
6. The compounds of Formulae IV and VIII, generally in the form of acetates, can be used as mineral enricheners either by the flotation technique of the mineral as well as to act as reagents for extraction with solvent.

A case is separation of sylvite (KCl) in the processes of potash production. Another case is the eliminaiton of Si0₂, which is separated by flotation of the foam, of ferrous minerals.

Another important case is the extraction with solvent of the uranium of the minerals that contain it. The uranimum mineral that contains U₃O₈ is treated in the first place with sulfuric acid. The acid liquor is extracted with kerosene containing 3-6% by weight of tertiary amine of formula II and VI preferably of C₆-C₈ chain and 1-4% by weight of fatty alcohol of an oxo nature. At selective pH the enrichment of the solution in uranyl sulfate is achieved. Finally it is separated in the ammoniated complex form for final processing thereof. Other minerals treated by this system are vanadium, molybdenum and cobalt.

In accordance with the above, the use of the above mentioned novel compounds in the following fields of use is also object of the present invention.
1. Preparation of formulations of household softeners and detergents-softeners and of cosmetic formulations for hair conditioners and skind conditioners.
2. Preparation of industrial textile auxiliaries for softening, antistatic treatment and dyeing of fibers and fabrics.
3. Preparation of disinfectant formulations wherein they act due to the bactericide, fungicide and algicide properties thereof.
4. Preparation of anticaking addtives of granular, powder-form or prill form fertilizers.
5. As cationic asphaltic emulsifiers and as improvers of adhesion of asphalt to substrates and also as acid corrosion inhibitors on ferrous material surfaces.
6. As mineral flotation agents (sylvinite flotation) and extraction with solvent (uranium extraction.)

With the use of the novel compounds described efficient performances for each use mentioned and also an improvement for the environment upon working with compounds with a high degree of biodegradability, at the level of the requirements of present regulations are achieved, exceeding in many cases the ecotoxicological limitations of the normally used products.

### EMBODIMENTS OF THE INVENTION

The present invention is additionally illustrated by means of the following Examples, which do not restrict the scope thereof.

### EXAMPLE 1 (Reaction 1, steps A and B)

190.2 g (1 mol) of N,N-dimethyl-N',N'-diethoxy-1,3-propanediamine are charged in a 2000 ml. equipped with a variable speed mechanical stirrer, thermometer, electric heating system, distillation column, refrigerant and collector. While a slight nitrogen current is passed, 547.4 g (2.02 mols) of tallow fatty acid are added kept above the melting point thereof. The reaction temperature, due to the heat from neutralization, rises alone up to 110º C and once the addition has ended the temperature is made to rise gradually up to 170-180ºC and it is kept for 5 more hours. The obtained product, after eliminating the reaction water contains 98% ester (Formula II) with a Gardner 2-4 color.

100 g. (0.11 mols) of the product thus obtained and 13 g. of isopropanol are charged in a 250 ml. flask provided with reflux refrigerants. The mixture is heated to 55º C and 18 g. (0.143 mols) of dimethyl sulfite are added, drop by drop and with continuous stirring and keeping the reaction temperature between 60-65º C for an interval of 4 hours. Thus, the corresponding compound of Formula I is obtained.

### EXAMPLE 2 (Reaction 1, steps A and B)

190.2 g (1 mol) of N,N-dimethyl-N',N'-diethoxy-1,3-propanediamine are charged in a 1000 ml. reactor equipped like in the previous example and 240 g. (1.2 mols) of lauric acid are added. It is heated to 170º C and kept for 3-4 hours, obtaining by elimination of the water a product of Formula IV.

100 g. (0.3 mols) of the product thus obtained and 15 g. of isopropanol are charged in a 250 ml. flask provided with reflux refrigerant. The mixture is heated to 55º C and 37 g. (0.29 mols) of benzyl chloride are added in the interval of 1 1/2 hours endeavoring that the temperature does not exceed 65º C for which purpose cooling must take place towards the end of the addition. After 3 hours the reaction is considered as ended. 15 more g. of water are added and a product of Formula III is obtained.

Another 100 g (0.3 mols) of the product obtained according to paragraph one and 250 g. of water are charged in a 1000 ml. reactor, provided with reflux refrigerant. The mixture is heated to 80º C and 34.6 g. of crystallized sodium monochloroacetate ared added without stopping the stirring, keeping the pH between 8,5 and 9.5 for 5 hours without the temperature exceeding 80º C. Thus, a compound of Formula II, of an amphoteric nature, is obtained.

### EXAMPLE 3 (Reaction 2, steps A and B)

102.2 g (1 mol) of N,N-dimethylamino-1,3-propanediamine are heated to 100º C under vacuum for 10 minutes to decompose any existing form of carbonate. They are cooled to 25º C and under vigorous stirring 172 g. (2 mols) of methyl acrylate are added, drop by drop, refrigerating to keep the temperature between 25 and 30º C. The mixture is kept under these conditions for 8-10 hours until the reaction is completed (determination of the content of primary and tertiary amine.) It is heated to 80º C for 1 hour to eliminate the unreacted methyl acrylate.

274 g. (1 mol) of the above compound are reacted at 100º C under a gentle vacuum to help to eliminate the methanol released during the reaction with 386 g. (1.98 mols) of a C₁₂-C₁₄ lauryl alcohol, in the presence of 0.5 g. of sodium methylate 30%. Gradually methanol is eliminated from the system, the reaction of transesterification taking place which can be considered as ended after 6 hours, obtaining a compound of Formula VI.

### EXAMPLE 4 (Reaction 3, steps A, B and C)

102.2 g (1 mol) of N,N-dimethylamino-1,3-propanediamine are charged in a 1000 ml. reactor and 271 g. (1 mol) of tallow fatty acid are added drop by drop, in an interval of 1/2 hour. It is heated to 170-180º C and kept for 5 hours. The reaction ends when the acid value is lower than 10.

273 g. (1 mol) of hydrogenated tallow fatty acid are charged in a 1000 ml reactor and 92.5 g. (1 mol) of epichlorohydrin are added slowly at a temperature between 80 and 90º C. After the addition that lasts 1/2 hour has ended, it is kept for two more hours until the acid value is lower than 10.

100 g. (0.28 mols) of the product obtained in the first paragraph are reacted with 102.3 g. (0.28 mols) of the product obtained in paragraph two, keeping the temperature between 60 and 70º C and a slight flow of nitrogen for two hours. The determination of total amine and of tertiary amine makes it possible to control the end of the reaction that leads to a compound of Formula IX.

### EXAMPLE 5 (Reaction 4, steps A and B)

195 g. (1 mol) of linear C₁₂-C₁₄ lauryl alcohol, to which 1.5 g. of sodium methylate in an alcohol solution 30%, are reacted at 60-70º C with 144 g. (1.01 mols) of N,N-dimethyl-N-(2-acryl-oxyethyl)amine added in the interval of 1 hour. The mixture is allowed to react for three more hours and then it is subjected to a vacuum (20 mm Hg) at 100º C to eliminate the residual monomer.

100 g. (0.296 mols) of the above compound are reacted at 60-70º C with 108 g. (0.296 mols) of the product resulting from the reaction of the hydrogenated tallow fatty acid with epichlorohydrin, as described in paragraph two of EXAMPLE 4. A slight nitrogen current is kept for two hours. The reaction is considered ended when the total amine value is zero. A compound of Formula X is obtained.

### EXAMPLE 6 (Diluted household softener formula)

| Component | Percentage by weight |
|---|---|
| Product of Formula I or V 90% (M = 820 - 840) | 5.0 |
| Preservative | 0.2 |
| Perfume | 0.3 |
| Dye (Soln.1 %) | 0.12 |
| Water | balance up to 100 g. |

Operation method: 50 g. of deionized water are heated to 60º C and 5 g. of quaternary product of Formula I or V melted, are added to water under vigorous stirring (200 rpm) until a total homogenous dispersion (stirring time 15-20 minutes.) After cooling to 25º C the dye, preservative and perfume are added. The viscosity of the final product is 20-30 cps, measured with a Brookfield LVT viscosimeter, spindle 1 at 60 rpm.

### EXAMPLE 7 (Diluted househould softener formula)

| Component | Percentage by weight |
|---|---|
| Product of Formula II or VI 100 % (M = 696 - 720) | 5.0 |
| HCl 35 % | 7.5 - 7.2 |
| Preservative | 0.2 |
| Dye (Soln. 1 %) | 0.12 |
| Water | balance up to 100 g. |

Operation method: It is identical to the previous example, charging in the first place the water and hydrochloric acid for the purpose of achieving a good dispersion of the aminic base.

### EXAMPLE 8 (Triple conc. household softener formula)

| Component | Percentage by weight |
|---|---|
| Product of Formula X 100 % (M = 763.5) | 17.0 |
| Preservative | 0.1 |
| Laurl alcohol + 8 MOE | 1.5 |
| Hexahydrate magnesium chloride | 0.2 |
| Dye (Aqueous soln. 1 %) | 0.36 |
| Perfume | 1.2 - 1.4 |
| Water | balance up to 100 g. |

Operation method: 60 g. of deionized water at 55º C are charged, to which the dye, the non-ionic derivative are added lsowly and under stirring (200-300 rpm), the previously melted cationic derivative. 0.1 g. of magnesium chloride are added and stirred for 20 minutes. The rest of the cold water is added and then it is cooled to 25º C. Then the preservative, 0.1 g. of magnesium chloride and the perfume are added. It is stirred for another 15 minutes and then it is discharged. The product has a viscosity between 40 and 90 cps which is ideal for direct use thereof in the final rinse in a washing machine or for intermediate dilution to the standard concentration by the housewife.

Formulations of concentrated softeners with contents of the cationic derivative or of amine salt up to 25% of the active principle, with the compounds of different Formulae I, II, V, VI, IX and X, can be obtained, especially varying the amounts of electrolyte and non-ionic component and even the type of the latter. In some cases the viscosities can be varied at will between 40 and 400 cps.

### EXAMPLE 9 (Enrichened hair conditioner formula)

| Component | Percentage by weight |
|---|---|
| Product of Formula III or VII with C₁₆ or C₁₆-C₁₈ chain quaternized with methyl chloride, dimethyl sulfate or benzyl chloride | 2.5 |
| Cationic polymer Polyquaternium 7 type according to CFTA designation | 2.5 |
| C₁₆-C₁₈ fatty alcohol | 2.0 |
| Sorbitan oleate + 20 mols O.E. | 1.0 |
| Propyleneglycol | 2.0 |
| Perfume, dye and preservative | q.s. |
| Deionized water | balance up to 100 g. |

Operation method: The first five components are charged in the same vessel. They are heated to 60º C, keeping the temperature and an inert atmosphere with nitrogen to prevent the increase of coloration. When the entire mass is melted it is homogenized well and then it is added slowly to another vessel that contains the water, also at 60º C. Once the addition has ended it is stirred slowly and the temperature drops itself to 35-40º C in about two hours. Just then the preservative, the dye and the perfume are added.

### EXAMPLE 10 (Low irritation shampoo formula)

| Component | Percentage by weight |
|---|---|
| Product of Formula III or VII with a totally hydrogenated C₁₂-C₁₈ chain. Structure with an amphoteric nature (reaction described with ClCH₂-COONa) (30% active) | 7.0 |
| Sodium laurylether sulfate 70% | 10.0 |
| Sodium hemisulfosuccinate 40% | 5.0 |
| Coconut diethanolamine | 2.5 |
| EDTA Na₂ | 0.5 |
| Preservative, dye and perfume | q.s. |
| Citric acid (to adjust the pH 6-6,5) NaCl to adjust the viscosity to 2500-3000 cps | approx. 1.5 |
| Deionized water | balance up to 100 g. |

Operation method: Half of the water at 45-50º C, the EDTA and the sodium laurylether sulfate 70% is charged and stirring is maintained until all the ingredients are added. Then, the other surface active agents are added. Once they have been included, the rest of the cold water is added. The pH is adjusted. The preservative, dye and perfume are added and finally the viscosity is adjusted.

### EXAMPLE 11 (Textile lubricant for cellulose, acrylic and polyester fibers)

| Component | Percentage by weight |
|---|---|
| Product of Formula I or III, with an oleic chain, quaternized with dimethyl sulfate. 85% active | 1.5 |
| Product of Formula II or IV, with partially hydrogenated tallow chain | 7.5 |
| Paraffin with m.p. 56-58 | 11.0 |
| Carnauba wax | 1.0 |
| C₁₆-C₁₈ linear fatty alcohol + 10-15 MOE | 1.0 |
| Formic acid 85% to adjust the pH 4-6 | q.s. |
| Preservative | q.s. |
| Deionized water | balance up to 100 g. |

Operation method: The paraffin, carnauba wax, the compound of Formula II or IV, the ethoxylated alcohol are charged and heated moderately to 90-95º C until perfect homogenization. The cationic derivative of Formula I or III is added and homogenized keeping the temperature and stirring of approximately 100 rpm. The pH is adjusted with formic acid dissolved in a small amount of deionized water. The remaining deionized water previously heated to 85-90º C is added slowly. At the end of the addition the pH is adjusted again, the preservative is added and stirring is maintained until the temperature drops by itself to 60-70º C at which time the product in the form of a translucid dispersion can be discharged.

The product can be obtained in the form of a paste up to 50% of concentration if the adequate amount of water is reduced.

### EXAMPLES 12 and 13 (Detergent/softener - softergent - formulae)

| Component | Percentage by weight | |
|---|---|---|
| | Example 12 | Example 13 |
| Sodium linear dodecylbenzene sulfonate 100 % | 8.0 | 8.0 |
| Sodium soap (80/20 tallow/coconut), powder | 1.0 | 1.0 |
| Oxo alcohol 13/15 + 7 MOE | 7.0 | 7.0 |
| STPP | 28.0 | 0.0 |
| Sodium zeolite, type A | 0.0 | 30.0 |
| Acrylic-maleic copolymer (35%) | 5.0 | 5.0 |
| Sodium silicate (SiO₂/Na₂O = 2) | 5.0 | 5.0 |
| Sodium perborate | 20.0 | 20.0 |
| TAED | 0.2 | 0.2 |
| Sodium carbonate | 1.5 | 6.0 |
| Carboxymethylcellulose | 1.0 | 1.0 |
| Optical whitener | 0.3 | 0.3 |
| Perfume | q.s. | idem. |
| Granular enzymes | 0.5 | 0.5 |
| Product of Formula III or VII, quaternized with dimethyl sulfate 35 % | 4.0 | 4.0 |
| Product of Formula II or VI with hydrogenated C₁₆-C₁₈ chain, 100 % | 4.0 | 4.0 |
| Sodium sulfate | balance up to 100 g. | |

The formulations described are alternatives for washing with hot water or with cold water though formulation 13 does not contain phosphates. Keeping the proportionality and reducing the sodium sulfate, they are valid for compact detergents. Both are likewise valid for powdered detergent and detergent in tablet form.

### EXAMPLE 14 (Detergent/softener formula in liquid form)

| Component | Percentage by weight |
|---|---|
| Product of Formula I, II, V, IX or X (85-100%) | 8.0 |
| Sodium laurylether sulfate (70%) | 20.0 |
| Sodium dodecylbenzene sulfonate, triethanolamine salt (100%) | 10.0 |
| Sulfonated alpha olefin, sodium salt, C₁₄-C₁₆ chain (35%) | 10.0 |
| Oxo alcohol C₁₃-C₁₅ + 7 MOE | 20.0 |
| Acrylic-maleic copolymer (35%) | 3.0 |
| Propyleneglycol | 4.0 |
| Ethanol | 0.5 |
| Enzymes | 0.2 |
| Citric acid | 0.2 |
| Monoethanolamine to adjust pH 8-9 | q.s. |
| Dye, flatting agent, optical whitener and perfume | q.s. |
| Water | balance up to 100 g. |

## Claims

1. Novel polyfunctional cationic surface active agents, characterized in that they have the following structural formulae: 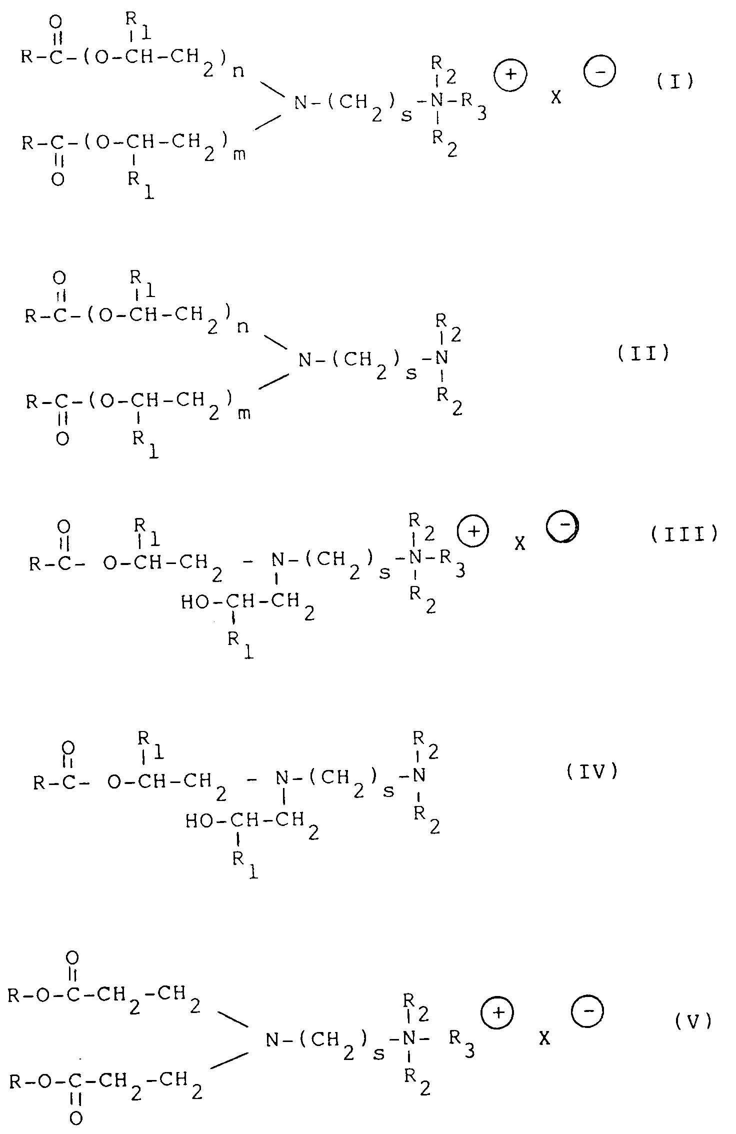 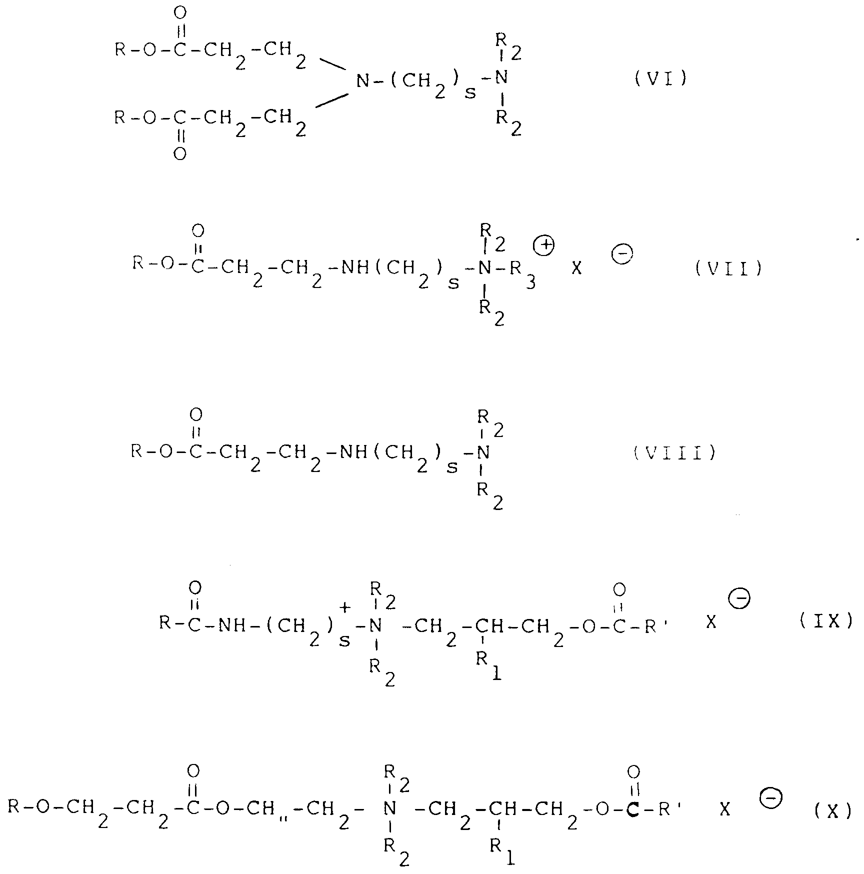 wherein:
R and R' represent alkyl, alkylaryl or alkoxyalkyl radicals, wherein the alkyl residue can be linear or branched and contains from 6 to 24 carbon atoms;
R₁ represents hydrogen or hydoxyl;
R₂ and R₃ represent a benzyl, alkyl or oxyalkyl group, wherein the alkyl residue contains from 1 to 6 carbon atoms;
X^{⊖} represents a Cl^{⊖}, SO₄CH₃^{⊖} or SO₄C₂H₅^{⊖} anion
n and m represent an integer comprised between 1 and 5, both inclusive; and
s represents an integer selected between 2 and 3.

2. Process for the preparation of novel polyfunctional cationic surface active agents of formulae: 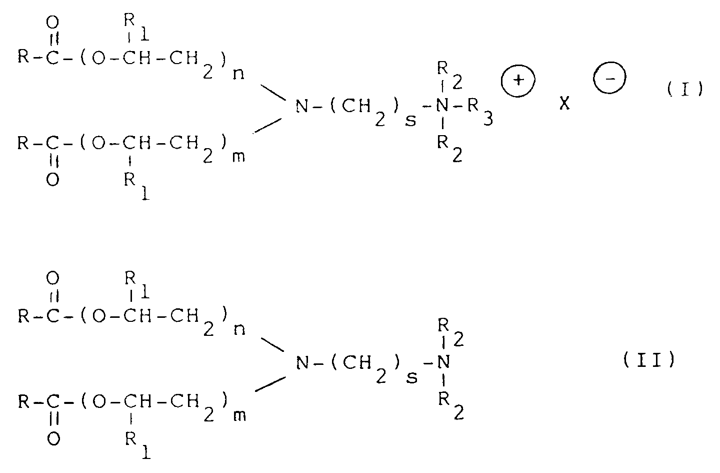 wherein:
R represents alkyl, akylaryl or alkoxyalkyl radicals, wherein the alkyl residue can be linear or branched and contains from 6 to 24 carbon atoms;
R₁ represents hydrogen or hydroxyl;
R₂ and R₃ represent a benzyl, alkyl or oxyalkyl group, wherein the alkyl residue contains from 1 to 6 carbon atoms;
X^{⊖} represents a Cl^{⊖} , SO₄CH₃^{⊖} or SO₄C₂H₅^{⊖} anion;
n and m represent an integer comprised between 1 and 5, both inclusive; and
s represents an integer selected between 2 and 3;
whose process is characterized in that it comprises:
a) reacting a compound of formula: 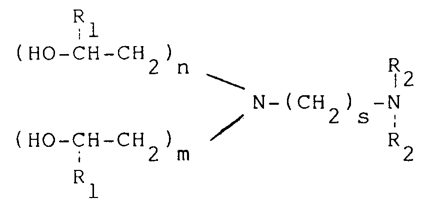 with a monocarboxylic acid of formula:
R-COOH
or an ester thereof, wherein the different symbols have the meaning given above, using a proportion of 2 mols of the cited acid or ester thereof per mol of N,N-dialyl-N'N-dipolyalkoxy-1,s-alkanodiamine at a temperature between 120 and 200º C in the presence of an acid catalyst, to produce the corresponding compound of formula (II)
b) optionally, reacting the compound of formula (II) thus obtained with an alkylating agent of formula X-R₃ to obtain the corresponding quaternary ammonium compound of above cited formula (I.)

3. Process for the preparation of novel polyfunctional cationic surface active agents of formulae: 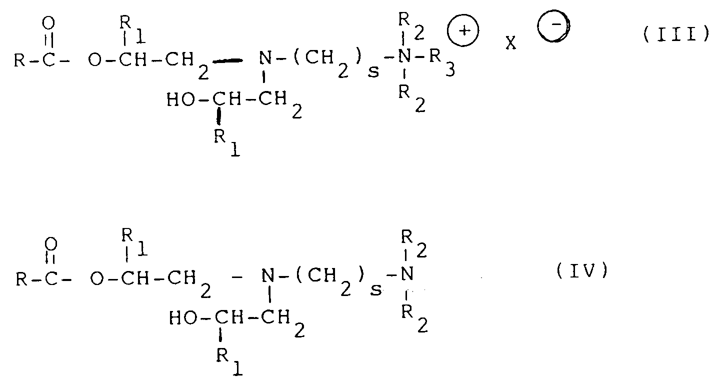 wherein:
R represents alkyl, alkylaryl or alkoxyalkyl radicals, wherein the alkyl residue can be linear or branched and contains from 6 to 24 carbon atoms;
R₁ represents hydrogen or hydroxyl;
R₂ and R₃ represent a benzyl, alkyl or oxyalkyl group, wherein the alkyl residue contains from 1 to 6 carbon atoms;
X^{⊖} represents a Cl^{⊖} , SO₄CH₃^{⊖} or SO₄C₂H₅^{⊖} anion;
n and m represent an integer comprised between 1 and 5, both inclusive; and
s represents an integer selected between 2 and 3;
whose process is characterized in that it comprises:
a) reacting a compound of formula: 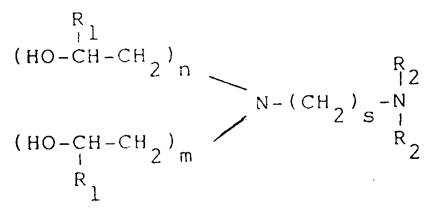 with a monocarboxylic acid of formula:
R-COOH
or an ester thereof, wherein the different symbols have the meaning given above, using a proportion of 1 mol thereof per each mol of N,N-dialkyl-N'N-dipolyalkoxy-1,s-alkanodiamine to obtain the corresponding compound of formula (IV);
b) optionally, reacting the compound of formula (IV) thus obtained with an alkylating agent of formula X-R₃ to obtain the corresponding quaternary ammonium compound of above cited formula (IV);
c) optionally, reacting the compound of formula (III) thus obtained with sodium monochloroacetate in an aqueous medium at 60-90º C and an alkaline pH so that carboxymethylation takes place obtaining the corresponding compound of an amphoteric nature of formula (I) wherein R₃ = carboxymethyl.

4. Process for the preparation of novel polyfunctional cationic surface active agents of formula: 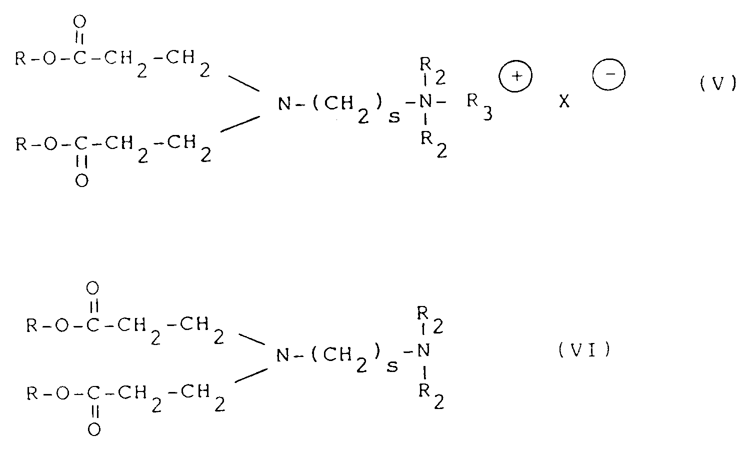 wherein:
R represents alkyl, alkylaryl or alkoxyalkyl radicals, wherein the alkyl residue can be linear or branched and contains from 6 to 24 carbon atoms;
R₁ represents hydrogen or hydroxyl;
R₂ and R₃ represent a benzyl, alkyl or oxyalkyl group, wherein the alkyl residue contains from 1 to 6 carbon atoms;
X^{⊖} represents a Cl^{⊖}, SO₄CH₃^{⊖} or SO₄C₂H₅^{⊖} anion;
n and m represent an integer comprised between 1 and 5, both inclusive; and
s represents an integer selected between 2 and 3;
whose process is characterized in that it comprises:
a) reacting two mols of methyl acrylate of formula
CH₂=CH-COOCH₃
with a mol of N,N-dialkyl-1,s-alkanodiamine of formula: 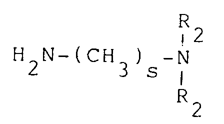 wherein s and R₂ have the meaning given above at a temperature comprised between 25 and 60º C, to produce an intermediate product of formula:
(CH₃OOC-CH₂-CH₂)₂N-(CH₂)ₛ-N(R₂)₂
wherein the different symbols have the meaning given above;
b) reacting the intermediate product thus produced, at a temperature of 100-120ºC with 2 mols of a fatty alcohol of formula: R-OH
wherein R is as defined above, with the simultaneous elimination of 2 mols of methanol, to produce the compound of formula (VI) indicated above;
c) optionally, reacting the compound of formula (VI) thus obtained with an alkylating agent of formula X-R₃ to obtain the corresponding quaternary ammonium compound of formula (V) indicated above.

5. Process for the preparation of novel polyfunctional cationic surface active agents of formulae: 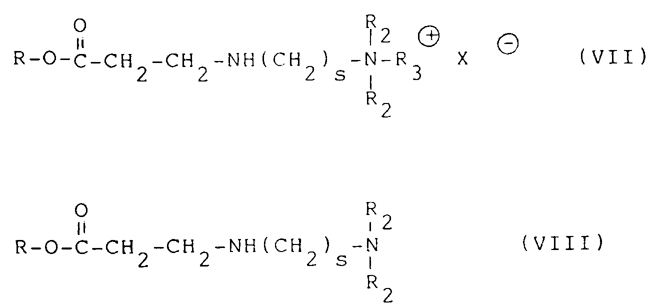 wherein:
R and R' represent alkyl, alkylaryl or alkoxyalkyl radicals, wherein the alkyl residue can be linear or branched and contains from 6 to 24 carbon atoms;
R₁ represents hydrogen or hydroxyl;
R₂ and R₃ represent a benzyl, alkyl or oxyalkyl group, wherein the alkyl residue contains from 1 to 6 carbon atoms;
X^{⊖} represents a Cl^{⊖}, SO₄CH₃^{⊖} or SO₄C₂H₅^{⊖} anion,
n and m represent an integer comprised between 1 and 5, both inclusive;
s represents an integer selected between 2 and 3;
whose process is characterized in that it comprises:
a) reacting 1 mol of methyl acrylate of formula:
CH₂=CH-COOCH₃
with a mol of a N,N-dialkyl-1,s-alkanodiamine of formula: 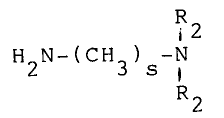 wherein s and R₂ have the meaning given above, at a temperature between 25 and 60º C to produce an intermediate product of formula:
CH₃OOC-CH₂-CH₂-NH(CH₂)ₛ-N(R₂)ₛ
wherein the different symbols have the meaning given above;
b) reacting the intermediate product thus produced at a temperature of 100-120º C with one mol of a fatty alcohol of formula:
R-OH
wherein R is as defined above, with the simultaneous elimination of 1 mol of methanol, to produce the compound of formula (VIII) indicated above;
c) optionally, reacting the compound of formula (VIII) thus obtained with an alkylating agent of formula X-R₃ to obtain the corresponding quaternary ammonium compound of formula (VII) indicated above;
d) optionally reacting the compound of formula (VII) thus obtained with sodium monochloroacetate in an aqueous medium and at an alkaline pH so that the corresponding carboxymethylation takes place thus obtaining the corresponding amphoteric derivative.

6. Process for the preparation of novel polyfunctional cationic surface active agents of formula (IX): 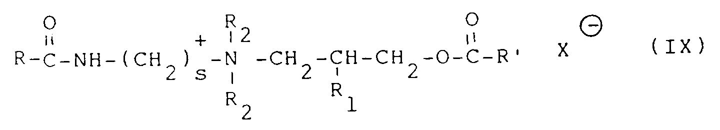 wherein:
R and R' represent alkyl, alkylaryl or alkoxyalkyl radicals, wherein the alkyl residue can be linear or branched and contains from 6 to 24 carbon atoms;
R₁ represents hydrogen or hydroxyl;
R₂ and R₃ represent a benzyl, alkyl or oxyalkyl group, wherein the alkyl residue contains from 1 to 6 carbon atoms;
X^{⊖} represents a Cl^{⊖}, SO₄CH₃^{⊖}, or SO₄C₂H₅^{⊖} anion;
n and m represent an integer comprised between 1 and 5, both inclusive; and
s represents an integer selected between 2 and 3;
whose process is characterized in that it comprises:
a) reacting a carboxylic acid with a chain of 6 to 24 carbon atoms of formula:
R-COOH
with a N,N-dialkyl-1,s-alkanodiamine of formula: 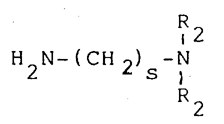 wherein the different symbols have the meaning given above, carrying out the reaction at a temperature of 130-200º C with elimination of the water that is formed during the course of amidation to produce a compound of formula: 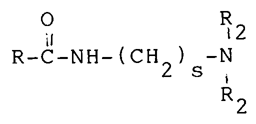 wherein the different symbols have the meaning given above;
b) reacting the amide thus obtained with a glyceric chloroester of formula: 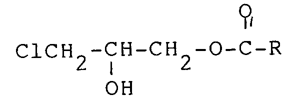 wherein R has the meaning given above to produce the compound of formula (IX) indicated above.

7. Process for the preparation of novel polyfunctional cationic surface active agents of formula (X) 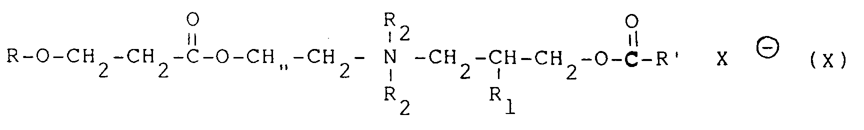 wherein:
R and R' represent alkyl, alkylaryl or alkoxyalkyl radicals, wherein the alkyl residue can be linear or branched and contains from 6 to 24 carbon atoms;
R₁ represents hydrogen or hydroxyl;
R₂ and R₃ represent a benzyl, alkyl or oxyalkyl group wherein the alkyl residue contains from 1 to 6 carbon atoms;
X^{⊖} represents a Cl^{⊖}, SO₄CH₃^{⊖} or SO₄C₂H₅^{⊖} anion;
n and m represent an integer comprised between 1 and 5, both inclusive; and
s represents an integer selected between 2 and 3;
whose process is characterized in that it comprises:
a) reacting a N,N-dialkyl-N-(2-acryloxyethyl)amine of formula: 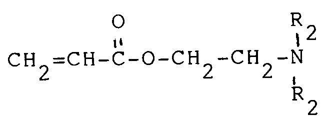 wherein R₂ has the meaning given above, with one mol of a linear or branched fatty alcohol whose chain can contain between 8 and 20 carbon atoms of formula R-OH, or a temperature of 50-80º C to produce a compound of formula: 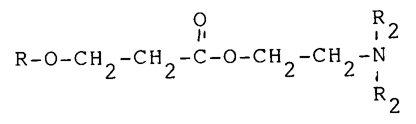 wherein R and R₂ have the meaning given above;
b) reacting the product thus obtained with a glyceric chloroester of formula: 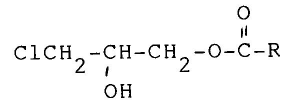 wherein R has the meaning give above, to produce the compound of formula (X) already indicated.

8. Fabric softener composition for cotton and synthetic fibers in aqueous dispersion form characterized in that it comprises:
- 1-40% by weight one one or more of the cationic surface active agents of formulae (I), (II), (V), (VI) (IX) and (X) indicated in claim 1, that can optionally be combined with any other conventional cationic surface active agent;
- 0-10 % by weight of a monohydric C₁-C₄ monohydric alcohol or a C₂-C₆ glycol;
- 0-10% of a non-ionic surface active agent;
- 60-90% by weight of a solvent vehicle;
- 0-10% by weight of fatty acids;
along with other optional ingredients present in minor amounts, selected from among preservatives, dyes, siloxanes, inorganic salts and perfumes.

9. Hair conditioner composition, characterized in that it comprises:
- 0.5-10% by weight of one or more of the cationic surface active agents of formulae (I) to (X) indicated in claim 1;
- 0.10% by weight of a saturated linear chain fatty alcohol;
- 0-5% by weight of cationic polymers CFTA;
- 0-5% by weight of non-ionic surface active agents;
- 0-5% by weight of emollients;
along with other optional ingredients present in minor amounts selected from among dyes, preservatives and perfumes.

10. Use of the compounds of formulae (I) to (X) defined in claim 1, in the manufacture of softener and detergent-softener formulations of household use, as well as cosmetic formulations to condition hair and skin.

11. Use of the compounds of formulae (I) to (X) defined in claim 1, in the manufacture of industrial textile auximiliaries for softening, antistatic treatment and dyeing of fibers and fabrics.

12. Use of the compounds of formula (I), (III), (V) and (VII) defined in claim 1, in the manufacture of disinfectant formulations with bactericide, fungicide or algicide properties.

13. Use of the compounds of formulae (IV) and (VIII) defined in claim 1, in the preparation of anti-caking additives for granular, powder-form or prill form fertilizers.

14. Use of the compounds of formulae (IV) and (VIII) defined in claim 1, as cationic asphaltic emulsifiers and as improvers of adhesion of alphalt to substrates and also as acid corrosion inhibitors on the surface of ferrous materials.

15. Use of the compounds of formulae (IV) and (VIII) defined in claim 1, as mineral enrichener auxiliaries either by the flotation technique or else by the extraction with solvent technique.
